# EUROPÄISCHE PATENTSCHRIFT

(11) **EP 1 140 215 B1**
(45) Veröffentlichungstag und Bekanntmachung des Hinweises auf die Patenterteilung: **15.06.2005**
(21) Anmeldenummer: 99948753.1
(22) Anmeldetag: 14.09.1999
(51) Int. Cl.: A61L 2/04, C02F 1/02

(54) **VORRICHTUNG ZUM THERMISCHEN STERILISIEREN VON FLÜSSIGKEITEN**
DEVICE FOR THE THERMAL STERILIZATION OF LIQUIDS
DISPOSITIF DE STERILISATION THERMIQUE DE LIQUIDES

(43) Veröffentlichungstag der Anmeldung: 10.10.2001
(73) Patentinhaber: Biermaier, Hans, 86316 Derching (DE)
(72) Erfinder: Biermaier, Hans, 86316 Derching (DE)
(74) Vertreter: von Bülow, Tam, Dr.
(86) Internationale Anmeldenummer: PCT/EP1999/006812
(87) Internationale Veröffentlichungsnummer: WO 2001/019412

(56) Entgegenhaltungen:
- EP-A- 0 061 779
- EP-A- 0 214 589
- EP-A- 0 814 312
- DE-A- 3 627 578
- DE-C- 19 837 923
- US-A- 4 203 205

## Beschreibung

Die Erfindung bezieht sich auf eine Vorrichtung zum thermischen Sterilisieren von Flüssigkeiten, insbesondere von Trinkwasser, gemäß dem Oberbegriff des Patentanspruches 1.

Eine derartige Vorrichtung zur Entkeimung von Trinkwasser ist aus der DE 195 22 234 A1 bekannt. Diese Vorrichtung weist eine Pumpe auf, die zu sterilisierendes Wasser ansaugt und durch den Heizabschnitt eines Gegenstromwärmetauschers in einen Boiler pumpt, wo es erhitzt wird, um Keime bzw. Bakterien abzutöten. Das erhitzte Wasser wird von dem Boiler zurück durch den Kühlabschnitt des Wärmetauschers gepumpt und dort unter Wärmeabgabe an das angesaugte Wasser im Heizabschnitt auf Gebrauchstemperatur abgekühlt.

In der DE 31 19 632 A1 ist eine Sterilisationsanlage für Milch beschrieben, die einen Milchkreislauf und einen hiervon getrennten Wärmteträgerkreislauf aufweist, wobei die beiden Kreise durch zwei gemeinsame Wärmetauscher thermisch miteinander gekoppelt sind.

Aus der DE 42 00 588 A1 ist eine Vorrichtung zur Heißsterilisierung pumpfähiger Lebensmittel bekannt, die im wesentlichen aus einem horinzontal angeordneten spiralförmigen Rohr besteht, das zur Hälfte mit Lebensmittelbrei und zur Hälfte mit Heißdampf gefüllt wird. Durch Drehen des spiralförmigen Rohres wird der Lebensmittelbrei gefördert und sterilisiert.

Die DE 40 03 987 C2 beschreibt einen Wärmetauscher mit zwei Flüssigkeitskreisläufen, der zur thermischen Sterilisierung eingesetzt wird.

Aus der DE 39 25 795 A1 ist ein Spiralrohrwärmetauscher mit einem zylindrischen Gehäuse bekannt, das einen tangential angeordneten Einströmkanal für ein erstes Medium aufweist, dessen Querschnitt durch einen Drosselschieber variierbar ist. Im Gehäuse ist ein spiralförmiger Kanal angeordnet, der von einem zweiten Medium durchströmt wird. Das einströmende erste Medium strömt zunächst an der Gehäusewand entlang und dann zwischen die einzelnen Windungen der Spirale bis in deren Zentrum und dort durch einen Auslaßkanal aus dem Gehäuse heraus.

Die DE 32 02 587 A1 beschreibt einen Spiralwärmetauscher der aus Keramik hergestellt ist, aus drei voneinander getrennten Strömungskanälen besteht und für einen Wärmetausch zwischen Rauchgasen und Flüssigkeiten vorgesehen ist.

Ein ähnlicher Spiralwärmetauscher ist in der DE 29 21 841 beschrieben, der einen spiralig verlaufenden Kanal für den Durchfluß eines zu kühlenden Mediums, und zwei ebenfalls spiralig verlaufende getrennte Kühlkanäle aufweist.

Aus der DE 35 09 226 C2 ist ein Spiralwärmetauscher bekannt, der ein zylindrisches Gehäuse aufweist, in den ein spiralförmig gebogener Blechstreifen eingesetzt ist, der die Strömungskanäle für die wärmetauschenden Medien trennt.

In der DE 31 22 947 A1 sind mehrere Spiralwärmetauscher beschrieben, deren Rohre an den Berührflächen miteinander verlötet sind. Diese Wärmetauscher weisen verschiedene Rohrquerschnitte und verschiedene Spiralformen auf, wie z.B. Spirale mit kreisförmiger, rechteckiger, achteckiger oder kegelförmiger Kontur.

Aus der DE 33 19 521 C2 ist ein Spiralwärmetauscher bekannt, dessen spiralförmige Kanäle in zwei durch eine Trennwand getrennten Gehäusehälften angeordnet sind. Das zu kühlende Medium strömt durch einen Einlaß der einen Gehäusehälfte, von dort in einem spiralförmigen Kanal nach innen in eine Kammer im Zentrum des Gehäuses, von der Kammer in die zweite Gehäusehälfte und von dort zu einem entsprechenden Kanal zu einem Auslaß der zweiten Gehäusehälfte. In umgekehrter Weise strömt das Kühlmedium durch spiralförmige Kanäle von der zweiten in die erste Gehäusehälfte.

Aufgabe der Erfindung ist es, eine Vorrichtung zur thermischen Sterilisierung von Flüssigkeiten, insbesondere von Trinkwasser zu schaffen, die einfach und kostengünstig herstellbar, kompakt aufgebaut ist und einen geringen Energieverbrauch hat.

Diese Aufgabe wird durch die Merkmale des Patentanspruches 1 gelöst. Vorteilhafte Ausgestaltungen und Weiterbildungen der Erfindung sind den Unteransprüchen zu entnehmen.

Der Erfindung liegt die Idee zugrunde, zur thermischen Sterilisierung von Flüssigkeiten einen Gegenstromwärmetauscher zu verwenden, in dessen Zentrum eine Heizquelle angeordnet ist.

Die Heizquelle ist von einer spiralartig angeordneten Leitung umgeben, durch welche die zu sterilisierende Flüssigkeit strömt. Die Leitung ist vorzugsweise durch flexible Folien, sei es aus Metall oder Kunststoff, gebildet und "spiralartig" um die Wärmequelle herumgewickelt. Der Begriff "spiralartig" ist hier nicht streng mathematisch zu verstehen, sondern bedeutet, daß die einzelnen Leitungswindungen "übereinanderliegend" angeordnet sind und einander berühren, d.h. die Leitungswindungen können im Querschnitt - ähnlich wie bei einer mathematischen Spirale -in einer Ebene liegen oder kugelförmig um die Heizquelle herum angeordnet sein. Die Leitung hat dabei zwei Strömungskanäle, nämlich einen Zulaufkanal zur Wärmequelle, den Heizabschnitt des Wärmetauschers und einen Abflußkanal von der Wärmequelle, den Kühlabschnitt des Wärmetauschers. Da sich die einzelnen Leitungswindungen berühren, steht, außer bei der äußersten und der innersten Windung, jeder Bereich des Heizabschnittes beidseitig mit einem Kühlabschnitt und jeder Bereich des Kühlabschnittes beidseitig mit einem Heizabschnitt in wärmetauschendem Kontakt. Dabei wird die im Heizabschnitt strömende Flüssigkeit unter Wärmetausch mit der im Kühlabschnitt strömenden Flüssigkeit vorgewärmt, durch die Heizquelle auf Sterilisationstemperatur erhitzt und anschließend im Kühlabschnitt abgekühlt.

Die spiralartige Anordnung der Leitung wird bei Verwendung einer Leitung aus elastischem, d.h. leicht biegbaren Material einfach durch "Umwickeln" der Heizquelle erreicht und ermöglicht eine optimale, d.h. schnelle und verlustarme Wärmeübertragung auf die zu sterilisierende Flüssigkeit. Durch den kontinuierlichen Wärmetausch zwischen dem abzukühlenden sterilisierten Heißwasser und dem zugeführten zu sterilisierenden Kaltwasser erreicht man somit eine relativ hohe Vorwärmtemperatur, so daß zur Erreichung der für die Sterilisierung erforderlichen Maximaltemperatur eine relativ geringe Heizquellenleistung genügt. Ferner wird das sterilisierte Wasser durch den Wärmetausch wieder annähernd auf Gebrauchstemperatur abgekühlt und kann daher sofort verwendet werden.

Das Umwickeln der Heizquelle mit der elastischen Leitung hat ferner den Vorteil, daß die Vorrichtung sehr kompakt herstellbar ist und daß selbst größere Flüssigkeits- bzw. Dampfdrücke, die nahe der Heizquelle, d.h. in "inneren" Leitungswindungen auftreten, auf alle Windungen verteilt werden, was die Verwendung kostengünstiger elastischer Leitungsmaterialien ermöglicht. Eine besonders gleichmäßige Verteilung des Flüssigkeitsdrucks und gleichzeitig eine sehr kompakte Bauweise läßt sich durch eine kugelförmige Anordnung der Leitung erreichen. Durch die Elastizität der Leitung ist ferner sichergestellt, daß sich einzelne Leitungswindungen bei einer dampf- oder wasserdruckbedingten Dehnung innig berühren und ein guter Wärmeübergang erreicht wird.

Da in der Nähe der Heizquelle Temperaturen von 100 bis 150 °C erreicht werden und damit das Wasser lokal verdampft, wird der dabei kurzfristig entstehende Überdruck von der flexiblen Leitung über den gesamten Warmetauscher verteilt. Dabei werden aufgrund der Flexibilität der Leitung die Kanäle mit dem relativen Überdruck aufgeweitet und die Kanäle mit dem relativ hierzu niedrigeren Druck verengt, wodurch eine Art Peristaltikbewegung entsteht, die - zusätzlich zum Wasserdruck am Eingang des Wärmetauschers - den Weitertransport des Wassers fördert. In Verbindung mit einem Rückschlagventil am Einlaß erhält man so einen stoß- oder impulsweisen Wasserdurchfluß durch den Wärmetauscher mit Phasen hoher und niedriger Strömungsgeschwindigkeit. Aufgrund dieses "Pumpeffektes" arbeitet die Vorrichtung auch mit minimalsten Wasserdruck am Eingang des Wärmetauschers. Durch die phasenweise hohe Strömungsgeschwindigkeit wird einem Verschmutzen und Verkalken des Wärmetauschers vorgebeugt.

Im Heizabschnitt ist eine Einrichtung, wie z.B. ein Rückschlagventil, vorgesehen, die eine Flüssigkeitsströmung nur in Richtung zum Kühlabschnitt ermöglicht und eine Rückströmung zu einer Wasserversorgung, beispielsweise zu einem Leitungsnetz, an den der Heizabschnitt der Vorrichtung angeschlossen ist, verhindert. Durch das Erhitzen des Wassers auf z.B. 100 oder 150°C entstehen nämlich in der Leitung Drücke von mehreren Bar, die größer sein können als der Wasserdruck im Leitungsnetz, wobei das Rückschlagventil ein Rückströmen verhindert. Das Rückschlagventil schließt solange, bis der Wasserdruck in der Leitung durch das Abfließen des Wassers aus dem Kühlabschnitt auf bzw. unter den Wasserdruck im Leitungsnetz abgesunken ist, so daß frisches zu sterilisierendes Wasser nachströmen kann. Das Rückschlagventil ist vorzugsweise am Zulauf des Heizabschnitts angeordnet, d.h. unmittelbar an der Anschlußstelle zur Wasserversorgung.

Die Vorrichtung gemäß der Erfindung ist vielfältig einsetzbar, da lediglich eine Energieversorgung, wie z.B. ein Stromanschluß für die Heizquelle und ein Wasseranschluß benötigt werden, wobei ein Betrieb auch bei relativ geringen Wasserdrücken möglich ist. Dies ermöglicht beispielsweise einen Einsatz in privaten Haushalten, Kliniken, als Vorschaltgerät zu Maschinen aber auch in Entwicklungsländern, wo Trinkwasser kostengünstig vorort sterilisiert werden muß.

Nach einer Weiterbildung der Erfindung besteht die Leitung aus zwei elastischen Folien, die an ihren Längsrändern miteinander verschweißt sind. Alternativ dazu ist es auch möglich, die Leitung aus drei an ihren Längsrändern miteinander verschweißten Folienlagen herzustellen, wobei jeweils ein Ende der beiden äußeren Folien an einem Ende der Leitung ebenfalls miteinander verschweißt ist. Als Material können beispielsweise flexible Metall- bzw. Kunststoffolien verwendet werden. Alternativ dazu ist es auch möglich, als Leitung zwei ineinandergeschobene elastische Rohre, insbesondere Kapillarrohre bzw. Schläuche zu verwenden, wobei die beiden Leitungsabschnitte durch den Ringraum zwischen den beiden Rohren und das innere Rohr gebildet sind. Auch können parallel laufende Rohre bzw. Schläuche verwendet werden, die durch das "Wickeln" ebenfalls in gutem thermischen Kontakt zueinander stehen.

Im folgenden wird die Erfindung anhand von Ausführungsbeispielen näher erläutert. Es zeigen:
- Fig. 1a: ein erstes Ausführungsbeispiel einer Vorrichtung gemäß der Erfindung;
- Fig. 1b: ein zweites Ausführungsbeispiel einer Vorrichtung gemäß der Erfindung;
- Fig. 2a: einen Querschnitt durch eine Leitung, die aus zwei miteinander verschweißten Folien besteht;
- Fig. 2b: den Übergangsbereich zwischen dem ersten und zweiten Leitungsabschnitt der Leitung der Fig. 2a;
- Fig. 3a: eine Leitung, die aus drei miteinander verschweißten Folien besteht;
- Fig. 3b: den Übergangsbereich der Leitung der Fig. 3a;
- Fig. 4a: eine Leitung die aus zwei Rohren besteht; und
- Fig. 4b: den Übergangsbereich der Leitung der Fig. 4a.

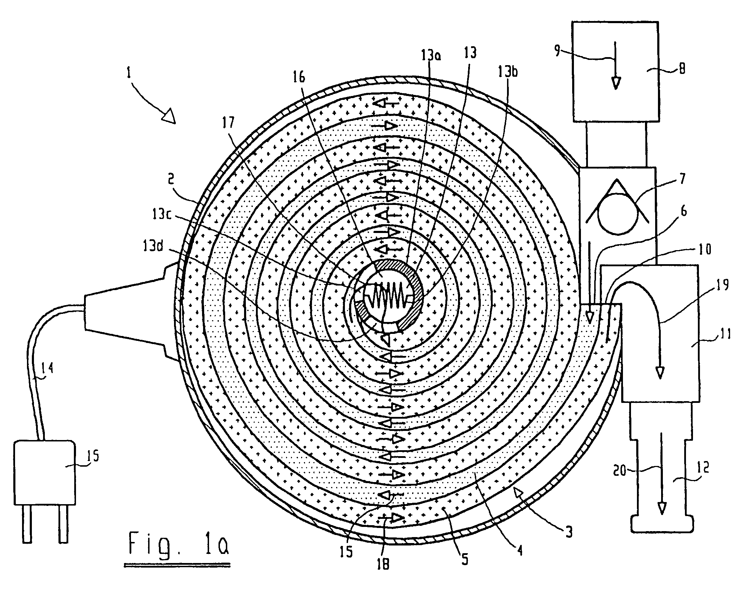

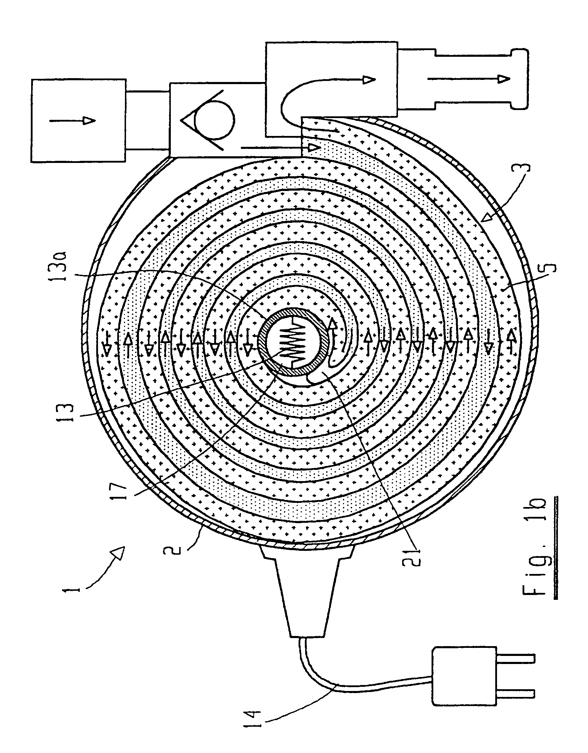

Fig. 1a zeigt eine Vorrichtung 1 zum thermischen Sterilisieren von Flüssigkeiten, bestehend aus einem zylindrischen Gehäuse 2, in dem eine zu einer Spirale gewickelte Leitung 3 angeordnet ist, die einen Heizabschnitt 4 und einen Kühlabschnitt 5 aufweist. Am Zulaufende 6 des Heizabschnitts 4 ist ein Rückschlagventil 7 und ein Anschlußstück 8 vorgesehen, mit dem die Vorrichtung an eine Wasserversorgung wie z.B. an einen Wasserhahn angeschlossen werden kann. Das Rückschlagventil 7 ermöglicht ein Zuströmen von Wasser in den Heizabschnitt 4 in der durch den Pfeil 9 angegebenen Richtung und verhindert ein Rückströmen zur Wasserversorgung. Am Austrittsende 10 des Kühlabschnitts 5 ist ein Anschlußstück 11 mit einem offenen Austrittsstück 12 vorgesehen, aus dem das Wasser ungehindert abfließen kann bzw. an das ein Schlauch, eine Maschine etc. angeschlossen werden kann.

In der Mitte des Gehäuses 2 ist eine Heizvorrichtung 13 vorgesehen, die beispielsweise elektrisch beheizt ist und hier aus einem zylindrischen Heizquellengehäuse 13a und einer darin angeordneten Heizwendel 13b besteht, die über ein aus dem Gehäuse 2 herausgeführtes Anschlußkabel 14 bzw. über einen Stecker 15 mit Strom versorgt wird. Das Heizquellengehäuse 13a weist eine Eintrittsöffnung 13c für den Eintritt von zu erhitzendem Wasser auf, die mit dem Heizabschnitt 4 verbunden ist und eine Austrittöffnung 13d für den Austritt erhitzten Wassers, die mit dem Leitungsabschnitt 5 verbunden ist. Selbstverständlich ist der Heizwendel 13b gegenüber der vorbeiströmenden Flüssigkeit elektrisch isoliert.

Wasser, daß über das Anschlußstück 8 und das Rückschlagventil 7 in den Heizabschnitt 4 eintritt, fließt bis zur Mitte der "Leitungsspirale" in der durch Pfeile 15 gekennzeichneten Richtung und wird durch das im Kühlabschnitt 5 rückfließende Wasser allmählich erwärmt. In einem Bereich 16, im Inneren des Heizquellengehäuses 13a erreicht das Wasser seine Maximaltemperatur und fließt dann - was durch Pfeile 17 und 18 dargestellt ist - in der "Leitungsspirale" zum Ende 10 des Kühlabschnitts 5 nach außen. Dort fließt das Wasser über das Anschlußstück 11 und das Austrittsstück 12 ab, was durch Pfeile 19 und 20 dargestellt ist.

Aufgrund der innigen beidseitigen Berührung der Leitungsabschnitte 4 bzw. 5 wird das über den Heizabschnitt 4 einströmende Wasser durch das im Kühlabschnitt 5 ausströmende Wasser vorgewärmt und das im Kühlabschnitt 5 ausströmende Wasser wird durch das im Heizabschnitt 4 zuströmende Wasser abgekühlt. Über die einander berührenden Windungen des ersten und Kühlabschnitts 4 bzw. 5 erfolgt also ein "innerer Wärmetausch". Dies hat den Vorteil, daß das im Heizabschnitt 4 zuströmende Wasser in der innersten Windung der "Leitungsspirale" auf eine bereits relativ hohe Temperatur vorgewärmt ist und durch eine relativ geringe Heizqullenleistung auf die gewünschte Sterilisierungstemperatur erhitzt werden kann, die in dem Bereich 16 zwischen dem Heizabschnitt 4 und dem Kühlabschnitt 5 erreicht wird.

Alternativ zu dem gezeigten Ausführungsbeispiel, bei dem die Leitung 3 so gewickelt ist, daß die einzelnen Leitungswindungen im Querschnitt in einer Ebene liegen, ist auch eine im wesentlichen kugelförmige Anordnung der Leitung 3 möglich, bei der lediglich die Energiezufuhr zur Heizquelle, wie z.B. elektrische Leitungen, eventuell eine Abweichung von der Kugelform bedingen. Ferner kann anstatt des Anschlußstücks 8 und des Austrittsstücks 12 jeweils auch ein Muffenanschluß vorgesehen sein, über den die Vorrichtung an ein Rohrleitungssystem angeschlossen ist.

Fig. 1b zeigt ein Ausführungsbeispiel gemäß der Erfindung, bei dem im Unterschied zu Fig. 1a die Leitungsabschnitte 4 und 5 nicht an das Heizquellengehäuse 13a "angeschlossen" sind und das zu sterilisierende Wasser die Heizwendel 13b nicht unmittelbar umströmt. Hier wird die Wärme von der Heizwendel 13b durch Wärmeleitung über das Heizquellengehäuse 13a auf die hierauf aufgewickelte Leitung 3 bzw. das darin strömende Wasser übertragen, wobei ein Übergangsbereich zwischen dem Heizabschnitt 4 und dem Kühlabschnitt 5 mit 21 bezeichnet ist.

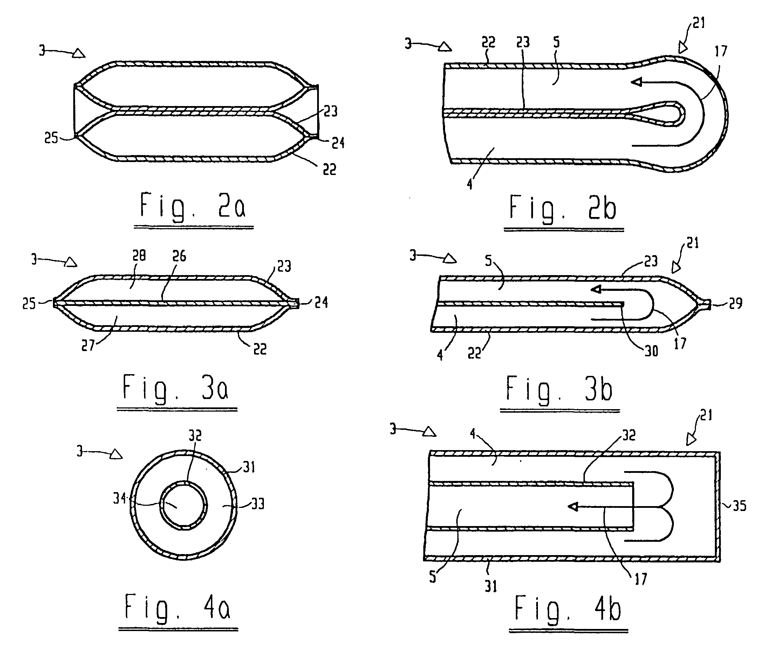

Die Fig. 2a, 3a und 4a zeigen Querschnitte durch die Leitung 3 und die Fig. 2b, 3b und 4b zeigen Längsschnitte durch den in Fig. 1b gezeigten Übergangsbereich 21 der Leitung 3.

Die in den Fig. 2a und 2b gezeigte Leitung besteht aus zwei Folien 22 bzw. 23, die in ihren Randbereichen 24 bzw. 25 miteinander verschweißt sind, wobei das Wasser in dem durch die beiden Folien 22 bzw. 23 gebildeten Hohlraum strömt, was in Fig. 2b durch den Pfeil 17 angedeutet ist. Im Übergangsbereich 21 (vgl. Fig. 1b) zwischen dem Heizabschnitt 4 und dem Kühlabschnitt 5 ist die Leitung 3 "umgeschlagen", so daß sich die beiden Leitungsabschnitte 4 bzw. 5 berühren, was einen guten Wärmeübergang ermöglicht. Die derart "umgeschlagene" Doppelleitung wird kann um die Wärmequelle herum spiralig gewickelt.

Die Fig. 3a und 3b zeigen ein weiteres Ausführungsbeispiel, bei dem die Leitung 3 aus drei elastischen Folien gebildet ist, nämlich den Folien 22 und 23 und einer dazwischen angeordneten Trennfolie 26. Entsprechend den Ausführungsbeispielen der Fig. 2a und 2b sind die Folien 22, 23 und 26 in ihren Randbereichen miteinander verschweißt. Ein erster Hohlraum 27, der zwischen der Folie 22 und der Trennfolie 26 liegt, bildet den Heizabschnitt 4 und ein zweiter Hohlraum 28, der zwischen der Folie 23 und der Trennfolie 26 liegt, bildet den Kühlabschnitt 5. Im Gegensatz zu dem in den Fig. 2a und 2b gezeigten Ausführungsbeispiel sind die Folien 22 und 23 an einem Ende 29, das den Übergangsbereich 21 zwischen dem Heizabschnitt 4 und dem Kühlabschnitt 5 bildet, miteinander verschweißt. Die Trennfolie 26 hat im Übergangsbereich 21 ein "freies Ende", was eine Flüssigkeitsströmung aus dem Heizabschnitt 4 in den Kühlabschnitt 5 entsprechend Pfeil 17 ermöglicht.

Fig. 4a und 4b zeigen ein weiteres Ausführungsbeispiel, bei dem die Leitung 3 durch zwei ineinandergeschobene Rohre 31 bzw. 32 gebildet ist. Ein zwischen den beiden Rohren 31 und 32 liegender Ringraum 33 bildet den Heizabschnitt 4 (oder den Kühlabschnitt 5) und ein durch das innere Rohr 32 umschlossener Hohlraum 34 bildet den Kühlabschnitt 5 (oder den Heizabschnitt 4). Im Übergangsbereich 21 ist das Rohr 31 durch eine Endwand 35 verschlossen, während das Rohr 32 dort offen ist, so daß Wasser entsprechend der Richtung des Pfeils 17 vom Heizabschnitt 4 und dem Kühlabschnitt 5 fließen kann.

## Patentansprüche

1. Vorrichtung zur thermischen Sterilisierung von Flüssigkeiten, insbesondere von Wasser, mit einer Gegenstromwärmetauscher, der eine Leitung mit einem Heizabschnitt und einem Kühlabschnitt aufweist, die miteinander in Strömungsverbindung stehen, und mit einer Heizquelle zum Aufheizen der Flüssigkeit, wobei
der Heizabschnitt und der Kühlabschnitt spiralartig um die Heizquelle (13) herum angeordnet sind,
die Heizquelle (13) sich im wesentlichen im Zentrum der Spirale befindet,
die Leitung (3) aus flexiblem, wickelbarem Material besteht,
einzelne Windungen der Leitung (3) übereinander liegen und einander berühren, **dadurch gekennzeichnet,**
**daß** eine Einrichtung (7) vorgesehen ist, die eine Flüssigkeitsströmung nur in Richtung von dem Heizabschnitt (4) zu dem Kühlabschnitt (5) ermöglicht.

2. Vorrichtung nach Anspruch 1, **dadurch gekennzeichnet, daß** die Einrichtung ein Rückschlagventil (7) ist.

3. Vorrichtung nach einem der Ansprüche 1 oder 2,
**dadurch gekennzeichnet,**
**daß** die Einrichtung (7) auf dem Heizabschnitt (4) angeordnet ist.

4. Vorrichtung nach einem der Ansprüche 1 bis 3,
**dadurch gekennzeichnet,**
**daß** die Einrichtung (7) am Zulaufende (6) des Heizabschnitts (4) angeordnet ist.

5. Vorrichtung nach einem der Ansprüche 1 bis 4,
**dadurch gekennzeichnet,**
**daß** die Leitung (3) durch zwei elastische Folien (22, 23) gebildet ist, die an ihren Längsrändern (24, 25) miteinander verschweißt sind.

6. Vorrichtung nach einem der Ansprüche 1 bis 4,
**dadurch gekennzeichnet,**
**daß** die Leitung (3) durch drei elastische Folien (22, 23, 26) gebildet ist, die an ihren Längsrändern (24, 25) miteinander verschweißt sind, wobei zwei (22, 23) dieser Folien durch die dritte Folie (26) voneinander getrennt sind.

7. Vorrichtung nach einem der Ansprüche 1 bis 4,
**dadurch gekennzeichnet,**
**daß** die Leitung (3) durch zwei ineinandergeschobene rohrartige Einzelleitungen (31, 32) gebildet ist.

8. Vorrichtung nach einem der Ansprüche 1 bis 7,
**dadurch gekennzeichnet,**
**daß** die einzelnen Windungen der Leitung (3) in einer Ebene liegen.

9. Vorrichtung nach einem der Ansprüche 1 bis 7,
**dadurch gekennzeichnet,**
**daß** die einzelnen Windungen der Leitung (3) kugelförmig angeordnet sind.

10. Vorrichtung nach einem der Ansprüche 1 bis 9,
**dadurch gekennzeichnet,**
**daß** die Leitung (3) aus einer Metallfolie oder einer Kunststofffolie besteht.

## Claims

1. A device for thermal sterilization of liquids, especially water, with a counterflow heat exchanger that has a conduit with a heating section and cooling section, which are in a flow connection to each other, and with a heating source for heating the liquid, in which
the heating section and the cooling section are spirally arranged around the heat source (13),
the heat source (13) is essentially located in the center of the spiral,
the conduit (3) consists of flexible, wrappable material,
individual windings of the conduit (3) lie one on the other and contact each other,
**characterized in that**
a fitting (7) that enables liquid flow only in the direction from the heating section (4) to the cooling section (5) is provided.

2. A device according to Claim 1, **characterized in that** the fitting (7) is a check valve.

3. A device according to Claims 1 or 2, **characterized in that**
the fitting (7) is arranged on the heating section (4) .

4. A device according to one of Claims 1 to 3, **characterized in that**
the fitting (7) is arranged at the inlet end (6) of the heating section (4).

5. A device according to one of Claims 1 to 4, **character** **ized in that**
the conduit (3) is formed by two elastic films (22, 23), which are welded to each other at their lengthwise edges (24, 25).

6. A device according to one of Claims 1 to 4, **characterized in that**
the conduit (3) is formed by three elastic films (22, 23, 26), which are welded to each other at their lengthwise edges (24, 25), where two (22, 23) of these films are separated from each other by the third film (26) .

7. A device according to one of Claims 1 to 4, **characterized in that**
the conduit (3) is formed by two tubular individual conduits (31, 32) arranged one inside the other in telescope fashion.

8. A device according to one of Claims 1 to 7, **characterized in that**
the individual windings of conduit (3) lie in the same plane.

9. A device according to one of Claims 1 to 7, **characterized in that**
the individual windings of the conduit (3) are arranged in a spherical form.

10. A device according to one of Claims 1 to 9, **characterized in that**
the conduit (3) consists of a metal film or a plastic film.

## Revendications

1. Dispositif de stérilisation thermique de liquides, notamment de l'eau, avec un échangeur thermique à contre-courant qui présente une conduite avec un segment de chauffage et un segment de refroidissement, lesquels sont en liaison d'écoulement l'un avec l'autre, et avec une source de chauffage servant à réchauffer le liquide, dans lequel
le segment de chauffage et le segment de refroidissement sont disposés en spirale autour de la source de chauffage (13),
la source de chauffage (13) se trouve essentiellement au centre de la spirale,
la conduite (3) est en matériau flexible enroulable,
les spires individuelles de la conduite (3) sont superposées et en contact les unes des autres, **caractérisé en ce que**
l'on prévoit un système (7) qui permet un écoulement de liquide seulement dans la direction partant du segment de chauffage (4) vers le segment de refroidissement (5).

2. Dispositif selon la revendication 1, **caractérisé en ce que** le système est un clapet anti-retour (7).

3. Dispositif selon l'une des revendications 1 ou 2, **caractérisé en ce que**
le système (7) est monté sur le segment de chauffage (4).

4. Dispositif selon l'une des revendications 1 à 3, **caractérisé en ce que**
le système (7) est monté à l'extrémité d'alimentation (6) du segment de chauffage (4).

5. Dispositif selon l'une des revendications 1 à 4, **caractérisé en ce que**
la conduite (3) est constituée par deux feuilles élastiques (22, 23) qui sont soudées ensemble sur leurs bords longitudinaux (24, 25).

6. Dispositif selon l'une des revendications 1 à 4, **caractérisé en ce que**
la conduite (3) est constituée par trois feuilles élastiques (22, 23, 26) qui sont soudées ensemble sur leurs bords longitudinaux (24, 25), deux (22, 23) de ces feuilles étant séparées l'une de l'autre par la troisième feuille (26).

7. Dispositif selon l'une des revendications 1 à 4, **caractérisé en ce que**
la conduite (3) est constituée par deux conduites individuelles tubulaires (31, 32) emboîtées l'une dans l'autre.

8. Dispositif selon l'une des revendications 1 à 7, **caractérisé en ce que**
les spires individuelles de la conduite (3) se trouvent sur un même plan.

9. Dispositif selon l'une des revendications 1 à 7, **caractérisé en ce que**,
les spires individuelles de la conduite (3) sont disposées en forme de sphère.

10. Dispositif selon l'une des revendications 1 à 9, **caractérisé en ce que**,
la conduite (3) est composée d'une feuille métallique ou d'une feuille en matière plastique.
